# EUROPEAN PATENT APPLICATION

(11) **EP 4 512 773 A1**
(43) Date of publication of application: **26.02.2025**
(21) Application number: 23791944.4
(22) Date of filing: 21.04.2023
(51) Int. Cl.: C01B 39/46, B01J 29/89, B01J 37/04, B01J 37/08, C01B 39/06, C01B 39/48, C07B 61/00, C07C 37/00, C07C 37/60, C07C 39/08, C07C 39/10

(54) **PRODUCTION METHOD FOR MODIFIED ALUMINOSILICATE, PRODUCTION METHOD FOR CATALYST INCLUDING MODIFIED ALUMINOSILICATE, PRODUCTION METHOD FOR AROMATIC POLYHYDROXIDE COMPOUND USING SAID CATALYST, AND MODIFIED ALUMINOSILICATE**

(30) Priority: 22.04.2022 JP 2022071046; 17.01.2023 JP 2023005412
(71) Applicant: Mitsui Chemicals, Inc., Tokyo 104-0028 (JP)
(72) Inventor: IMOTO, Itsuki, Sodegaura-shi Chiba 299-0265 (JP); NAKANISHI, Keita, Sodegaura-shi Chiba 299-0265 (JP); AKIYAMA, Satoshi, Sodegaura-shi Chiba 299-0265 (JP); HORIUCHI, Nobuhiko, Sodegaura-shi Chiba 299-0265 (JP); SAITO, Susumu, Sodegaura-shi Chiba 299-0265 (JP); KUBOTA, Yoshihiro, Yokohama-shi Kanagawa 240-8501 (JP); INAGAKI, Satoshi, Yokohama-shi Kanagawa 240-8501 (JP)
(74) Representative: Mewburn Ellis LLP
(86) International application number: PCT/JP2023/015884
(87) International publication number: WO 2023/204296

(57) **Abstract**

Provided is a method for producing a modified aluminosilicate capable of highly selectively producing hydroquinones by a reaction of phenols and hydrogen peroxide under industrially advantageous conditions, a method for producing a catalyst for producing an aromatic dihydroxide compound, the catalyst containing a modified aluminosilicate, a method for producing an aromatic dihydroxide compound using the catalyst, and a modified aluminosilicate.

The method for producing an aluminosilicate of the present invention includes a first step of preparing liquid in which sol-like silica containing water was contacted with a metal compound (AL) containing aluminum and oxygen to obtain an aluminosilicate having a specific range of a molar ratio of water to silica (HMR); a second step of treating the aluminosilicate obtained in the first step with an acid; a third step of subjecting the treated product obtained in the second step to primary calcination; and a fourth step of contacting the calcined product obtained in the third step with liquid containing one or more elements selected from the group consisting of elements in Group 4 and Group 5 of the Periodic Table, followed by carrying out drying and secondary calcination. The metal compound (AL) is preferably a zeolite. The first step preferably includes a step of removing water.

## Description

### Technical Field

The present invention relates to a method for producing a modified aluminosilicate, a method for producing a catalyst containing a modified aluminosilicate, a method for producing an aromatic dihydroxide compound using the catalyst, and a modified aluminosilicate.

### Background Art

Aromatic dihydroxide compounds are of importance as various organic synthesis intermediates or raw materials, and they are used as, for example, reducing agents, rubber chemicals, dyes, pharmaceuticals, agrochemicals, polymerization inhibitors, and oxidation inhibitors.

Aromatic dihydroxide compounds obtained by reacting phenols with hydrogen peroxide are, for example, hydroquinone and catechol, and production ratios of hydroquinone and catechol vary depending on production methods. In recent years, due to a demand balance between hydroquinone and catechol, a method of producing particularly hydroquinone with high selectivity has been increasingly desired.

In order to produce aromatic dihydroxide compounds by reacting phenols with hydrogen peroxide, a method for using a titanosilicate that is one of crystalline porous silicates, as a catalyst, has been disclosed (for example, Patent Literature 1 and Patent Literature 2). Moreover, Patent Literature 3 discloses a titanosilicate obtained by treating an acid-treated aluminosilicate with titanium chloride or titanium alkoxide in a gas phase.

Further, Patent Literature 4 discloses a method for producing a titanosilicate, which is obtained by mixing an aluminosilicate template material, an aluminum source, a titanium source, a silicon source, iodide, and water to prepare gel, which is then heated for crystallization and then calcinated.

The present inventors have disclosed a method for producing an aluminotitanosilicate by contacting an aluminosilicate compound with a liquid halogenated titanium compound (Patent Literature 5).

### Citation List

### Patent Literature

[Patent Literature 1] JP4254009
[Patent Literature 2] WO2015/041137
[Patent Literature 3] JP2008-050186A
[Patent Literature 4] JP2017-057126A
[Patent Literature 5] WO2019/225549

### Summary of Invention

### Technical Problem

The aluminotitanosilicate obtained in Patent Literature 5 suggests that it can be an excellent catalyst for producing an aromatic dihydroxide compound. The aforementioned literature discloses that among them, the aluminotitanosilicate with a titanium source introduced by using titanium tetrachloride gives an aromatic dihydroxide compound with particularly high selectivity. Moreover, according to an X-ray crystal structure analysis (X-ray diffraction (XRD) pattern) by the present inventors, the aluminotitanosilicate is a compound that exhibits a maximum peak at 23° to 24°.

According to investigations conducted by the present inventors, it has been found that a massy solid material may be generated in gel-like silica used as an intermediate raw material. From an industrial perspective, such a solid present may cause a problem in stably and quantitatively transferring the contents upon transferring them to a reaction apparatus (for example, an autoclave) for hydrothermal synthesis in the next step, and has a possibility to reduce, for example, reactivity due to being solid in a subsequent step. For this reason, it may be disadvantageous from the viewpoint of stable production. Pulverization of the solid material can practically solve the above problem, but carrying out the pulverization requires a special apparatus that leads to an increase in fixed costs and reduction of production speed, which may complicate a manufacturing step for industrial production.

An object of the present invention is to provide a method for producing a modified aluminosilicate such as an aluminotitanosilicate, capable of producing hydroquinones preferably with higher selectivity and a higher yield, for example, by reacting phenols with, for example, hydrogen peroxide, under more industrially advantageous conditions than before. The other object of the present invention is also to provide a catalyst capable of stably and highly selectively producing hydroquinones, for example, by reacting phenols with, for example, hydrogen peroxide under more industrially advantageous conditions, a method for producing an aromatic polyhydroxide compound using the catalyst, and a modified aluminosilicate.

### Solution to Problem

The present inventors have found as a result of investigations on the aforementioned objects that a silicate source such as silica in sol state containing water was contacted with an aluminum source such as a zeolite, in sol state, and thereafter a molar ratio of water to silica (H₂O/SiO₂) (hereinafter may be referred to as "HMR") was controlled, as a result of which the HMR value greatly affects performance of solid material described above. Providing a step of controlling the HMR value within a specific range has been found to allow a suitable aluminosilicate to be efficiently produced even from sol state which has better flowability than a gel. Via an acid treatment step or a contact treatment step with a transition metal compound such as a titanium compound, this aluminosilicate has also been found to be able to be converted into a modified aluminosilicate such as aluminotitanosilicate capable of producing hydroquinones with extremely high selectivity, then to complete the present invention.

That is, the present invention includes the following items [1] to [10].
[1] A method for producing a modified aluminosilicate, comprising
   a first step of preparing liquid in which sol-like silica containing water was contacted with a metal compound (AL) containing aluminum and oxygen to obtain an aluminosilicate in which a molar ratio of water to silica (H₂O/SiO₂: HMR) is in a range of 0.1 to 8.0,
   a second step of treating the aluminosilicate obtained in the first step with an acid,
   a third step of subjecting the treated product obtained in the second step to primary calcination at 550°C to 850°C, and
   a fourth step of contacting the calcined product obtained in the third step with liquid containing one or more elements selected from the group consisting of elements in Group 4 and Group 5 of the Periodic Table, followed by carrying out drying and secondary calcination.
[2] The method for producing a modified aluminosilicate according to [1], wherein the HMR is in the range of 3.0 to 6.6.
[3] The method for producing a modified aluminosilicate according to [1] or [2], wherein the metal compound (AL) containing aluminum and oxygen is a zeolite.
[4] The method for producing a modified aluminosilicate according to any one of [1] to [3], wherein the first step comprises a step of partially removing water.
[5] The method for producing a modified aluminosilicate according to [1], wherein the first step is a step of preparing liquid in which sol-like silica containing water was contacted with a zeolite, and then partially removing water in the liquid to obtain an aluminosilicate in which a molar ratio of water to silica (H₂O/SiO₂: HMR) is in a range of 0.1 to 8.0.
[6] A method for producing a catalyst for producing an aromatic polyhydroxide compound, the catalyst containing the modified aluminosilicate according to any one of [1] to [4].
[7] A method for producing a catalyst for producing an aromatic polyhydroxide compound, the catalyst containing the modified aluminosilicate according to [5].
[8] A method for producing an aromatic polyhydroxide compound, comprising a step of reacting an aromatic hydroxide with hydroperoxide in the presence of the catalyst according to [6].
[9] A method for producing an aromatic polyhydroxide compound, comprising a step of reacting an aromatic hydroxide with hydroperoxide in the presence of the catalyst according to [7].
[10] A modified aluminosilicate, having a ratio of an absorbance at 300 nm (A[300]) in an ultraviolet-visible absorption spectrum to an absorbance at 210 nm (A[210]) in an ultraviolet-visible absorption spectrum (A[300]/A[210]) of 0.045 or more and 0.070 or less.

### Advantageous Effects of Invention

According to the method for producing a modified aluminosilicate, one of the characteristics is to efficiently obtain an industrially useful aluminosilicate even though sol-like silica obtained, for example, by heating gel-like silica containing water, is used. In this manner, even sol-like silica containing water has been contacted with a metal compound (AL) containing aluminum and oxygen, a representative example of which is a zeolite (aluminum-containing compound crystal) that is an aluminum source, and then when a ratio of water to silica which are moieties of raw materials, is controlled within a specific range to carry out production, for example, an alminosilicate suitable for production of the aluminotitanosilicate which will be described below can be efficiently produced. The aluminosilicate is also preferably an aluminosilicate having the specific structure determined by an X-ray diffraction pattern. Even though the aluminosilicate is contacted with liquid containing one or more elements selected from the group consisting of elements in Group 4 and Group 5 of the Periodic Table, for example, an aqueous solution of a titanium source under mild conditions, an aluminotitanosilicate for use as a catalyst suitable for producing an aromatic polyhydroxide compound can also be obtained. Such an aluminotitanosilicate may also exhibit an ultraviolet-visible light spectrum (UV-Vis) that meets specific requirements. For this reason, the present invention is of industrial importance. Use of the modified aluminosilicate produced by the production method described above enables highly selectively producing an aromatic dihydroxide compound such as hydroquinone by a reaction of phenols with hydrogen peroxide.

### Brief Description of Drawings

FIG. 1 is a chart illustrating X-ray diffraction patterns of the aluminosilicates obtained in Examples 1 to 3 and Comparative Example 1.
FIG. 2 is a chart illustrating X-ray diffraction patterns of the aluminosilicate obtained in Examples 4 to 8.

### Description of Embodiments

Embodiments according to the present invention will be described in detail below.

### <Method for producing aluminosilicate>

The method for producing a modified aluminosilicate of the present invention includes a first step of preparing liquid in which sol-like silica containing water was contacted with a metal compound containing aluminum and oxygen (AL), preferably a zeolite to obtain an aluminosilicate in which a molar ratio of water to silica (H₂O/SiO₂: HMR) is in a range of 0.1 to 8.0,
a second step of treating the aluminosilicate obtained in the first step with an acid,
a third step of subjecting the aluminosilicate obtained in the second step to primary calcination at 550°C to 850°C, and
a fourth step of contacting the calcined product obtained in the third step with liquid containing one or more elements selected from the group consisting of elements in Group 4 and Group 5 of the Periodic Table, followed by carrying out drying and secondary calcination.

### [Sol-like silica]

As sol-like silica containing water, used in the present invention (hereinafter, the sol-like silica containing water is simply referred to as the "sol-like silica"), those publicly known can be used without limitation. The sol-like silica can be obtained, preferably by contacting a nitrogen-containing compound such as a quaternary ammonium salt or a known alkali source with a silica source such as commercially available colloidal silica and appropriately combining for use with heating and stirring.

Specific examples of the quaternary ammonium salts can include a salt of an ammonium ion (cation) containing 4 moles of a substituent such as a hydrocarbon group having 1 to 10 carbon atoms relative to 1 mole of nitrogen atoms, and an anion ion such as a hydroxy ion, or a halogen ion such as a chloride ion, a bromide ion or an iodide ion. The hydrocarbon group is more preferably a hydrocarbon group having 1 to 8 carbon atoms, further preferably 1 to 6 carbon atoms, and particularly preferably 1 to 4 carbon atoms. The anion is, on the other hand, more preferably a hydroxy ion, a bromide ion, and an iodide ion, and further preferably a hydroxy ion. More specific examples of such quaternary ammonium salts can include, for example, tetrapropylammonium hydroxide, tetrabutylammonium hydroxide, dimethyldipropylammonium hydroxide, tetrapropylammonium bromide, and bis-1,6-(tripropylammonium)hexamethylene diiodide. Particularly preferred is dimethyldipropylammonium hydroxide.

Such quaternary ammonium salts may be referred to as organic structure-directing agents, and may have the function of controlling the higher-order structure of silica or an aluminosilicate.

In the present invention, a ratio of the silica to the quaternary ammonium salt is preferably in a range of 1.5 to 10 as a silicon atom/nitrogen atom molar ratio. The lower limit value of the ratio is more preferably 1.7, further preferably 1.9 and particularly preferably 2.0. The upper limit value of the ratio is, on the other hand, more preferably 9, further preferably 8, and particularly preferably 7.

In an aspect of not using the seed crystal which will be described below, the silicon atom/nitrogen atom molar ratio may be preferably in a slightly higher range than the above range, and its preferred lower limit value is 2.5, more preferably 3.0, further preferably 3.6, and particularly preferably 3.7.

In the present invention, a molar ratio of water to silica in the sol-like silica is preferably a value exceeding 8.0.

Preferred examples of the water can include, for example, water contained in commercially available colloidal silica, water contained in the organic structure-directing agent described below or an alkaline source, and pure water combined for use in a method such as additional addition. Of course, other aspects than the aforementioned aspects may be used as long as water is used.

A temperature upon production of the sol-like silica is preferably a relatively elevated temperature. Such a temperature is preferably 40°C or higher, more preferably 50°C or higher, further preferably 60°C or higher, and particularly preferably 70°C or higher. The preferred upper limit temperature is 100°C, more preferably 95°C, further preferably 92°C, and particularly preferably 90°C.

Herein, the present invention is characterized in that liquid in which sol-like silica has been contacted with the metal compound (AL) is prepared and a molar ratio of water to silica (H₂O/SiO₂:HMR) is controlled in a range of 0.1 to 8.0. The control method preferably includes a step of partially removing water in liquid containing the sol-like silica and the metal compound (AL).

The lower limit value of the HMR is preferably 1.0, more preferably 2.0, further preferably 3.0, and particularly preferably 3.6. The upper limit value of HMR is, on the other hand, preferably 7.5, more preferably 7.0, further preferably 6.6, particularly preferably 6.2, and especially preferably 5.8. When the HMR is too low, a solid to be obtained may not obtain a sufficient effect in the second step (such as partial removal of aluminum by acid treatment) and the third step (such as introduction of transition metal by contact with a transition metal compound), which will be described below. The HMR exceeding 8.0 may not yield an aluminosilicate having the suitable configuration described above due to, for example, reduction of reaction rate.

Industrially, various steps are carried out using a plurality of reactors, and contents between the reactors are preferably transferred via piping. Therefore, various reactants are preferably in the form of particles having flowability, as well as in a liquid phase such as liquid, a solution, or a slurry. Conventionally, gel-like silica has been used as a raw material in the method of Patent Literature 5, but it is suitable for sol-like silica to be used as a silica raw material from the above viewpoint.

Such silica used, on the other hand, has been considered to have a possibility to result in an insufficient reaction with the metal compound (AL) and a possibility not to be able to obtain an effect of a structure-directing agent preferably combined for use and an effect of the seed crystal described below. However, the present inventors have found that as described before, under the conditions that an HMR at a stage of having contacted the sol-like silica with the metal compound (AL) meets a specific range, an aluminosilicate to be a suitable raw material of the catalyst described below can be obtained.

In the production method for obtaining an aluminosilicate having a specific structure as described below, according to the present invention, it is surmised that, for example, a shape and the number of pores of an aluminosilicate to be obtained are probably likely to be influenced by the HMR value, resulting in affecting conditions of reactions and forms of reaction products in the second step and the third step, which will be described below. For this reason, the present inventors think that it would be of importance to control the value within the above numerical range.

An effect of controlling the HMR in the previous manner is as described above, and may also significantly affect the second step and third step.

### [First step]

The first step is a step of preparing liquid in which sol-like silica that is a silicon source, is contacted with the metal compound (AL) that is an aluminum source to produce an aluminosilicate in which an HMR is in a range of 0.1 to 8.0. Examples of the metal compound (AL) can include known metal oxides such as various aluminum oxides and composite metal oxides including aluminum. More specific and preferred examples can include an aluminosilicate, with a zeolite being particularly preferred. The zeolite is preferably a FAU type zeolite and an MSE type zeolite and more preferably a FAU type zeolite. In this case, a step of preliminarily contacting an aluminosilicate crystal (A) having an MSE type structure with sol-like silica, is preferably included. Examples of the metal compound (AL) can also include aluminum salts such as aluminum hydroxide, sodium aluminate, aluminum nitrate, and aluminum sulfate.

The aluminosilicate obtained in the first step and the aluminosilicate crystal (A) are preferably porous. Hereinafter, the crystalline and porous aluminosilicate is simply referred to as a crystalline porous aluminosilicate.

The MSE-type aluminosilicate (A) can be considered to be a so-called "seed crystal" when producing the aluminosilicate described later. Use of crystals having the structure as described above may be advantageous in obtaining a modified aluminosilicate to be a raw material for a catalyst suitable for producing the aromatic polyhydroxide compound, which will be described below. On the other hand, it may be preferable not to use seed crystals, depending on production conditions.

The seed crystal material used in the present invention is not particularly limited as long as it has the above structure, but as described above, a crystalline porous aluminosilicate having an MSE-type structure (hereinafter referred to as "MSE framework") is preferred, and, for example, UZM-35, MCM-68, YNU-3 are particularly preferable, in the structure code of International Zeolite Association.

The crystalline porous aluminosilicate having an MSE framework has a portion in which a SiO₄ tetrahedron in which oxygen is arranged at the four vertices centered on silicon, and a TiO₄ tetrahedron in which titanium is arranged in place of silicon at the center are regularly bonded in a three-dimensional manner and has a three-dimensional porous structure having a 10-membered ring structure consisting of 10 units of the tetrahedrons and a 12-membered ring structure consisting of 12 units of the tetrahedrons, at their arbitrary proportions.

The first step of the present invention can be carried out by a method for contacting, for example, a known silicon source such as known colloidal silica, the metal compound (AL), preferably an aluminum source such as a zeolite, a quaternary ammonium salt (preferably an organic structure-directing agent such as dimethyldipropylammonium hydroxide) and a known alkali source, and stirring and heating the mixture. Incidentally, the silicon source, quaternary ammonium salt, and alkali source can include those derived from the sols.

A method for using gel-like silica as the silica source has been known, however, as described above, the gel-like silica becomes solid, and from the viewpoint of, for example, reaction efficiency, it may be difficult to obtain the effect in the second step and third step described below. From the viewpoint of flowability, the possibility is also considered of reducing productivity.

The present invention is characterized in that after contacted substances are obtained by contacting sol-like silica with the metal compound (AL), preferably an aluminum-containing compound such as zeolite, the molar ratio (HMR) of water to silica stays within a specific range. As described above, the present inventors have found that using an aluminosilicate obtained through such HMR conditions, provides a suitable product can be obtained when producing a modified aluminosilicate such as the aluminotitanosilicate and a catalyst, which will be described below. Any known method can be adopted to control the HMR within such a range. Examples thereof can include, for example, a method for heating the contacted substances to remove water under reduced pressure. The examples can also include a method for contacting the contact substances with a water-absorbing agent to remove water and more specifically a method for passing the contact substances through a fixed bed filled with a water-absorbing agent. Among the above methods, methods of heating, reduced pressure, and a combination of both, are suitable.

In the present invention, the first step preferably includes a step of partially removing water in liquid.

Incidentally, the HMR value can be specified using any known moisture content percentage measurement method, and can also be calculated from data related to a material balance in a reaction process.

A provided step in which the HMR falls within a specific range in the specific condition as described above makes it possible to efficiently produce a modified aluminosilicate such as aluminotitanosilicate and an aluminosilicate suitable as a raw material for a catalyst.

In order for the HMR to stay in the above range, a temperature at a stage of contacting sol-like silica with the metal compound (AL), preferably a zeolite, is, as in a preferred temperature range upon production of the sol-like silica, preferably 40°C or higher, more preferably 50°C or higher, further preferably 60°C or higher, and particularly preferably 70°C or higher. The preferable upper limit temperature is, on the other hand, 100°C, more preferably 95°C, further preferably 92°C, and particularly preferably 90°C.

Next, it is preferable that contacted substances such as the sol-like silica and the metal compound (AL), preferably a zeolite and preferably the seed crystal and a structure-directing agent, are heated to 140 to 175°C and held for 10 to 150 hours. The lower limit value of the temperature is more preferably 145°C and further preferably 148°C. The lower limit value may also be preferably 155°C, more preferably 157°C, further preferably 159°C, and particularly preferably 160°C. The upper limit value of the temperature is, on the other hand, more preferably 170°C, further preferably 168°C, and particularly preferably 165°C. The aforementioned conditions are preferable for producing a modified aluminosilicate such as the aluminotitanosilicate and an aluminosilicate to be a suitable raw material for a catalyst, which will be described below. From the viewpoints that, for example, the conditions described above can enhance selectivity of a produced compound, as disclosed in Examples, the conditions described above tend to be suitable. The above temperature range can be applied both when the seed crystal is used and when it is not used.

The lower limit value of the time is preferably 15 hours and more preferably 20 hours. The upper limit value is, on the other hand, preferably 140 hours and more preferably 130 hours. The time may be preferably 68 hours, more preferably 65 hours, and further preferably 60 hours.

In the present invention, for example, in an embodiment using the seed crystal, the heat treatment conditions may be preferably performed at a relatively low temperature. The low temperature preferably refers to 140°C or higher and lower than 160°C. The lower limit value of the temperature is preferably 145°C, and the upper limit value is preferably 157°C and more preferably 155°C. The lower limit value of the heat treatment time is preferably 10 hours, more preferably 20 hours, further preferably 23 hours, particularly preferably 30 hours, and especially preferably 35 hours. Depending on, for example, stirring efficiency and mixing efficiency of various components, derived from, for example, stirring conditions and a container shape, a longer time may be preferable, and for example, the lower limit value thereof is 40 hours, more preferably 45 hours, further preferably 50 hours, particularly preferably 55 hours, and especially preferably 60 hours. The upper limit value of the treatment time is preferably 140 hours and more preferably 130 hours. In an embodiment using such seed crystals, heating at low temperature may make it advantageous to obtain a modified alminosilicate that satisfies an ultraviolet light absorption intensity parameter in a special range as described below (for example, exhibits an excellent tendency in terms of a reaction yield).

In such a case, the HMR value is preferably 4.0 to 7.5. The lower limit value is more preferably 4.5, further preferably 5.0, particularly preferably 5.5, and especially preferably 5.7. The upper limit value is more preferably 7.0 and further preferably 6.8.

In the present invention, for example, in an embodiment of not using the seed crystal, the heat treatment conditions may be performed at a relatively low temperature for a long time. The low temperature refers to preferably 140°C or higher and lower than 165°C. The lower limit value of the temperature is preferably 145°C, and the upper limit value is preferably 160°C and more preferably 155°C. The long time refers to preferably 40 hours or longer and 150 hours or shorter. The lower limit value of the time is more preferably 50 hours, further preferably 60 hours, even further preferably 70 hours, particularly preferably 80 hours, and especially preferably 85 hours, and the upper limit value is, on the other hand, preferably 140 hours and more preferably 130 hours. In an embodiment of not using such a seed crystal, heating at low temperature for a relatively long time may make it advantageous to obtain a modified alminosilicate that satisfies an ultraviolet light absorption intensity parameter in a special range as described below (for example, exhibits an excellent tendency in terms of a reaction yield).

In such a case, the HMR value is preferably 3.0 to 6.0. The lower limit value is more preferably 3.5 and further preferably 4.0. The upper limit is, on the other hand, more preferably 5.8 and further preferably 5.6.

Carrying out the first step under the conditions as described above (for example, for the purpose of emphasizing reaction yield) tends to facilitate production of modified aluminosilicate with [A300]/[A210] within a specific range as described below. The reason for this tendency is currently unknown, however, the present inventors presume as follows.

It is considered that under the above conditions, an aluminosilicate proceedingly crystallizes from a sol state with its high degree of freedom to a condition in a slightly lower temperature, that is, in a relatively mild environment, as a result of which beautiful crystals are likely to be formed. However, since the reaction proceeds in a liquid phase at a relatively low temperature, a small amount of incomplete crystals may probably coexist. For this reason, the present inventors presume that the above method facilitates forming a form in which the [A300]/[A210] described below satisfies a specific range (0.045 to 0.070).

In an aspect using a seed crystal compared to a method not using the seed crystal, on the other hand, a condition in which the HMR is relatively high (the amount of water is large) is preferable. This can be presumed to be a tendency in which crystallization is promoted by the seed crystal, and therefore, in the method using the seed crystal, a condition of a relatively higher HMR, under which a silica component in a sol is considered to have a high degree of freedom, is considered to be preferable.

In a case in which the MSE-type aluminosilicate is used as a so-called seed crystal in the first step of the present invention, even though the MSE-type aluminosilicate is in a state of containing the sol-like silica, the HMR value falling within the above specific range exhibits an effect of promoting crystal growth upon synthesis of aluminosilicate, which is preferred. In the first step of the production method of the present invention, as sol-like silica is used, the step is carried out via a stage of at least initially contacting the silica with the metal compound (AL), preferably a zeolite, in a condition where a relatively large amount of water derived from the sol-like silica is present. It is also considered that the water has an effect of promoting a reaction with the metal compound (AL), preferably a component such as a zeolite, and in addition thereto, the water controlled so as to satisfy the above range of HMR, also has a possibility to exhibit an effect of promoting the crystal growth.

When using the seed crystal, the amount used is preferably 1 to 40% by weight and more preferably 2 to 30% by weight of silica as a silicon source. The metal compound (AL) that is an aluminum source, preferably a zeolite, also functions as a silicon source, and the amount used is preferably 5 to 50% by weight and more preferably 8 to 40% by weight of silica.

The aluminosilicate thus obtained preferably has the highest peak (β) in a region of 20° or larger and less than 22.5°in a region of 17.5 to 35° measured by XRD, and, a peak intensity ratio of the peak (β) to the highest peak (γ) in the region of 25 to 27° is preferably in a range of 1.60 to 5.0. The lower limit value of the peak intensity ratio is more preferably 1.65, further preferably 1.68, and particularly preferably 1.70. The upper limit value is, on the other hand, preferably 4.5 and more preferably 4.4.

Such an aluminosilicate used makes it possible to obtain, for example, a suitable catalyst for aromatic polyhydroxide compounds by using a method for introducing, for example, a titanium source as described below, the reason for which is unknown at this stage. Therefore, an aluminosilicate with a structure having the diffraction pattern as measured by XRD as described above is some preferred aspects of the present invention.

The aluminosilicate according to the present invention exhibits a crystal structure, but it is presumed that its crystal form is probably different from that of conventional aluminosilicates and is slightly unstable structure (in particular compared to a structure with the value of the peak intensity ratio of the peak (β) to the peak (γ)) or includes an unstable structure. It is also presumed that such instability may lead to excellent performance as a catalyst for producing aromatic polyhydroxide compounds when the modified aluminosilicate described below is formed.

Incidentally, when introducing the element of the present invention described below, which is selected from the group consisting of elements in Group 4 and Group 5 of the Periodic Table into a calcinated product of the aluminosilicate obtained via the second step and third step, which will be described below, an introduction site of the element can be considered to be mainly on a solid surface of the calcinated product of aluminosilicate, and therefore, the crystal structure may be considered to be almost the same before and after the introduction of the element.

Examples of raw materials for the sol-like silica include, for example, colloidal silica, sodium silicate, wet silica, and dry silica. These silicon sources can be used singly or in combinations of two or more thereof.

The metal compound (AL) serving as the aluminum source may be in combination of two or more thereof. Preferably, the metal compound (AL) is an aspect including a zeolite. As a component other than the zeolite, for example, a water-soluble aluminum compound can be used. Examples of the water-soluble aluminum compounds include, for example, aluminum hydroxide, sodium aluminate, aluminum nitrate, and aluminum sulfate.

As the alkali source, for example, a hydroxide containing an alkali metal can be used. Examples of hydroxides containing alkali metals include, for example, sodium hydroxide, and potassium hydroxide. These alkali sources can be used singly or in combinations of two or more thereof.

As the organic structure-directing agent, known compounds such as N,N,N',N'-tetraethylbicyclo[2,2,2]oct-7-ene-2,3:5,6-dipyrrolidinium diiodide and N,N,N',N'-tetraethylbicyclo[2,2,2]oct-7-ene-2,3:5,6-dipyrrolidinium diiodide can be used.

The aluminosilicate is preferably calcinated before the second step described below. The method of calcination is not particularly limited, and examples thereof include a method of calcination using, for example, an electric furnace and a gas furnace. The calcination conditions are preferably heating for 0.1 to 20 hours in an atmospheric atmosphere. A calcination temperature is preferably 500°C to 800°C and more preferably 550°C to 750°C.

### [Second Step]

The second step is a step of treating the aluminosilicate obtained in the first step with an acid, in other words, a step of contacting the aluminosilicate with the acid is the second step.

Examples of the acids used in this step include, for example, inorganic acids, organic acids, and mixtures thereof, and specific examples thereof include, for example, nitric acid, hydrochloric acid, sulfuric acid, citric acid, oxalic acid, and mixtures thereof. Among these, an acid containing an element selected from elements in Group 15 and Group 16 of the Periodic Table is preferable, and nitric acid is particularly preferable. A concentration of the acid is not particularly limited, but it is preferably 5% to 80% by weight and more preferably 40% to 80% by weight. When this acid is used as an aqueous solution, the amount thereof used is 1 to 100 parts by weight relative to 1 part by weight of an aluminosilicate. The lower limit value is more preferably 2 parts by weight, further preferably 3 parts by weight and particularly preferably 5 parts by weight. The upper limit value, is, on the other hand, more preferably 80 parts by weight, further preferably 70 parts by weight, particularly preferably 60 parts by weight, and especially preferably 50 parts by weight.

Temperature conditions for contacting the aluminosilicate with an acid are preferably 50°C to 170°C and more preferably 130°C to 170°C. A time of treatment with the acid is preferably 5 to 48 hours and more preferably 12 to 36 hours. The lower limit thereof is further preferably 18 hours. The contact with this acid is considered to partially remove aluminum from the aluminosilicate. Mainly aluminum on the surface of the aluminosilicate is presumed to be removed. Selecting the conditions of the relatively high temperature and long period of time as described above is presumed to facilitate forming a structure that is advantageous for introduction of an element in Group 4 and Group 5 in the calcination treatment in the third step described below.

### [Third step]

In the third step, the treated product obtained in the second step, that is, the aluminosilicate contacted with an acid is subjected to primary calcination. Calcination methods are not particularly limited, and include a method of using, for example, an electric furnace or a gas furnace. The calcination conditions are preferably heating for 0.1 to 20 hours in an atmospheric atmosphere. A calcination temperature is from 550°C to 850°C and more preferably from 600°C to 800°C. The primary calcination under this relatively high temperature environment is presumed to provide a favorable environment for formation of the so-called modified aluminosilicate obtained in the fourth step, which contains elements in Group 4 and Group 5 of the Periodic Table, with titanium species as representative example, in a highly active condition.

It is preferable that before calcining an aluminosilicate that has been contacted with the acid, the treated product is filtered using, for example, a Nutsche to separate, for example, the acid used (aqueous solution), and then a solid portion (filtrate) is washed with water and dried. The washing step described above is preferably carried out while maintaining a wet condition without drying before washing. A drying method after washing with water is not particularly limited, but it is preferable to uniformly and quickly dry it, and, for example, external heating methods such as hot air drying and superheated steam drying, and electromagnetic wave heating methods such as microwave heating and drying, and high frequency dielectric heating and drying, can be used.

### [Fourth step]

In the fourth step, the calcinated product obtained in the third step (hereinafter also referred to as a "primary calcinated product") and liquid containing one or more elements selected from elements in Group 4 and Group 5 of the Periodic Table are contacted in a liquid phase as element sources, followed by carrying out drying and secondary calcination. Examples of the elements in Group 4 and Group 5 of the Periodic Table can include, for example, titanium, zirconium, hafnium, and vanadium. The elements are preferably titanium, zirconium, and vanadium, with titanium being particularly preferred. Examples of the compounds containing these elements can include a halide, an alkoxide, or an inorganic acid salt of each element as a suitable compound. More preferably, the halides are chlorides, and alkoxides are aspects containing alkoxy groups having 1 to 6 carbon atoms. Examples of more preferred alkoxides include, for example, an ethoxide, a butoxide (n-butoxide, i-butoxide, s-butoxide, t-butoxide), and sulfate. These compounds for use may be in combinations of two or more thereof.

An aspect in which titanium is used as liquid containing titanium (titanium source in liquid phase), which is the most preferred aspect among elements in Group 4 and Group 5 of the Periodic Table, will be described as a representative example.

The titanium source in liquid phase is liquid containing titanium. Examples of the liquid containing titanium include, for example, a titanium compound in liquid form itself or an aqueous solution of a titanium compound. Among these, a titanium compound that is substantially acidic in its liquid condition is a preferred aspect.

Examples of titanium compounds in liquid form include, for example, titanium tetrachloride (TiCl₄), and titanium tetrabutoxide, with titanium tetrachloride being preferred among them. Examples of aqueous solutions of titanium compounds include, for example, a titanium tetrachloride aqueous solution, titanium trichloride (TiCl₃) aqueous solution, a titanium sulfate (Ti(SO₄)₂) aqueous solution, and a potassium hexafluorotitanate aqueous solution, with the titanium tetrachloride aqueous solution, titanium trichloride aqueous solution, and titanium sulfate aqueous solution being preferred among them. In the present invention, even when a titanium source having lower reactivity than titanium tetrachloride, such as titanium trichloride or titanium sulfate, is used other than titanium tetrachloride, the catalytic activity described below can be exhibited.

The liquid containing the titanium can be used singly or in combination of two or more thereof. For the liquid containing the titanium, a commercially available product can also be used, or liquid appropriately prepared by diluting a solid titanium compound with water to the desired concentration can also be used. Compared to a gas-phase titanium source, a liquid-phase titanium source (liquid containing titanium) is less likely to leak, which improves the problem of corrosion of, for example, production machinery and analytical equipment, as a result of which the liquid-phase titanium source can be expected to facilitate industrial production.

Conditions for contacting the primary calcinated product with the titanium source are not particularly limited, however, the contact allows titanium to be introduced into the primary calcinated product of the aluminosilicate. In this introduction of titanium, it is considered that there takes place a reaction of introducing titanium to the position of aluminum that was thought to have been removed in the second step, or a reaction of partially replacing aluminum in the aluminosilicate with titanium. As specific conditions for this step, for example, when using a titanium compound in liquid form itself, the titanium compound in liquid form is preferably added in an amount of 5 to 300 parts by weight per 1 part by weight of the primary calcined product and more preferably in an amount of 20 to 250 parts by weight. When using an aqueous solution of a titanium compound, the aqueous solution of a titanium compound is preferably added in an amount of 1 to 10 parts by weight per 1 part by weight of the primary calcined product and more preferably in an amount of 1 to 7 parts by weight. A concentration of the aqueous solution varies also depending on the compound used, but is, for example, 10 to 70% by weight and preferably 15 to 60% by weight.

As for the amount of titanium compound in the aqueous solution, the lower limit value thereof is preferably 0.1 g per 1 g of the primary calcined product, more preferably 0.2 g, further preferably 0.3 g, and particularly preferably 0.5 g. On the other hand, the upper limit value thereof is, preferably 10 g, more preferably 5 g, and further preferably 3 g.

Contact between the titanium source and the primary calcined aluminosilicate may be carried out only once, or each component may be used in multiple times, as long as each weight used is within a range of the amount added. For example, a titanium source may be added to the primary calcined product, and the calcined product obtained by carrying out drying and secondary calcination as described below may undergo drying and secondary calcination by adding a titanium source again. When adding the titanium source, hydrogen chloride is generated by a reaction between moisture in the air and a titanium compound, and therefore the reaction is preferably carried out under a nitrogen atmosphere.

Examples of more preferred specific methods can include, for example, a method for contacting the primary calcinated product with the titanium source, thoroughly mixing them, and then heat-treating the mixture, and a method for thoroughly drying them by the same method as the drying method exemplified in the third step, and then undergoing secondary calcination. A temperature in the heat treatment and drying treatment is not particularly limited, but, is preferably in a range of 20 to 150°C, for example, in order to effectively introduce titanium into the primary calcinated product. The lower limit value is more preferably 30°C, further preferably 40°C, and particularly preferably 50°C. On the other hand, the upper limit value is, more preferably 140°C, further preferably 120°C, and particularly preferably 100°C. A time required for the step is also not particularly limited, but is preferably 0.1 to 24 hours. The lower limit value is more preferably 0.3 hours, further preferably 0.4 hours, and particularly preferably 0.5 hours. On the other hand, the upper limit value is more preferably 12 hours and further preferably 6 hours. Methods of secondary calcination are not particularly limited, and, for example, an electric furnace or a gas furnace can be used for calcination. The calcination conditions are preferably 400°C or higher and 850°C for lower and from 0.1 to 20 hours in an atmospheric atmosphere. The lower limit value of the calcination temperature is more preferably 500°C, further preferably 550°C, and particularly preferably 600°C. On the other hand, the upper limit value is more preferably 800°C, further preferably 750°C, and particularly preferably 700°C.

Temperatures upon the primary calcination and the secondary calcination can be independently selected. A temperature upon the secondary calcination which is higher than that upon the primary calcination may be preferable.

Through this fourth step, for example, a crystalline porous aluminotitanosilicate that is a suitable aspect of the modified aluminosilicate of the present invention, in which aluminum in a crystalline porous aluminosilicate is thought to have been partially substituted with titanium, can be obtained.

Before performing the drying treatment, a mixture of the titanium source and the primary calcinated product may be heated to preliminarily remove moisture, filtered to remove impurities, and undergo, for example, washing operation with an organic solvent, followed by carrying out drying and secondary calcination.

The above production conditions can also be applied to a case where an element other than titanium is used.

Examples of compounds containing an element in Group 4 of the Periodic Table, which can be used in place of the titanium source include, for example, zirconium tetrachloride, tetraalkoxyzirconium, hafnium tetrachloride, tetraalkoxyhafnium, and zirconium sulfate, which can be combined for use with, for example, water, an alcohol and an ether, if necessary, to liquefy them for use. Examples of the liquefied compound include, for example, aqueous solutions of these compounds and solutions of alcohols and ethers. Examples of compounds containing an element in Group 5 of the Periodic Table, which can be used in place of the titanium source also include, for example, vanadium pentachloride, vanadium sulfate, vanadium trichloride, and an alkoxy-substituted derivative thereof, and they can be combined for use with, for example, water, an alcohol and an ether, if necessary, to liquefy them for use. Examples thereof include, for example, aqueous solutions of these compounds and solutions of alcohols and ethers.

### [Modified aluminosilicate]

The modified aluminosilicate obtained in the fourth step is preferably a crystalline porous aluminosilicate having crystallinity and porosity, as with the aluminosilicate that is the raw material, obtained in the first step, more preferably a crystalline porous aluminosilicate having an MSE framework, further preferably a crystalline porous aluminosilicate having UZM-35, MCM-68, or YNU-3 structure, and particularly preferably a crystalline porous aluminotitanosilicate. The crystallinity can be considered to be the same aspect as described for the aluminosilicate that is the raw material.

The crystalline porous aluminosilicate is a porous crystal at least partially having a portion in which a SiO₄ tetrahedron in which oxygen is arranged at the four vertices centered on silicone, and a AlO₄ tetrahedron in which aluminum is arranged in place of silicon at the center, are regularly bonded in a three-dimensional manner, and can be typically said to be one type of zeolite including an aluminosilicate. The modified aluminosilicate having crystallinity and porosity contains, for example, aluminum and one or more elements selected from the group consisting of elements in Group 4 and Group 5 of the Periodic Table in a crystalline porous aluminosilicate framework, and preferably it is obtained by such a method for partially replacing aluminum in the crystalline porous aluminosilicate framework with one or more elements selected from the group consisting of elements in Group 4 and Group 5 of the Periodic Table. As a more suitable example, when one or more elements selected from the group consisting of elements in Group 4 and Group 5 of the Periodic Table are titanium, the crystalline porous modified aluminosilicate is a crystalline porous aluminotitanosilicate and the crystalline porous aluminotitanosilicate contains aluminum and titanium in its aluminosilicate framework, and preferably it is obtained by such a method for replacing a portion of the aluminum in its framework by titanium.

The crystalline porous aluminosilicate having an MSE framework has a three-dimensional porous structure having a 10-membered ring structure consisting of 10 units of the tetrahedrons and a 12-membered ring structure consisting of 12 units of the tetrahedrons, at their arbitrary proportions. It is conjectured that pores with the 12-membered ring structure facilitate a substrate to diffuse inside the pores, making it easier to obtain high catalytic activity. Moreover, the absence of a large cavity inside the pore is considered to be one of factors that facilitates selectivity at the para position in an oxidation reaction of phenol.

It is well known that porous compounds have a wide specific surface area. The modified aluminosilicate of the present invention preferably has a specific surface area of 50 to 1000 m²/g. The lower limit value of its specific surface area is more preferably 100 m²/g and further preferably 150 m²/g. On the other hand, the upper limit value of its specific surface area is, more preferably 800 m²/g, and even more preferably 600 m²/g.

The value of the specific surface area can be determined by a known calculation method based on a BET theory by creating a BET plot from the measurement results using a known nitrogen adsorption/desorption measurement apparatus method (for example, BELSORP-max manufactured by MicrotracBEL Corp.).

The preferred range of pore volume of the modified aluminosilicate of the present invention is 0.1 to 0.5 cm³/g and more preferably 0.15 to 0.4 cm³/g.

The content of each element in Group 4 and Group 5 in the modified aluminosilicate of the present invention is not particularly limited. For example, when titanium is contained as one or more elements selected from the group consisting of elements in Group 4 and Group 5 contained in the modified aluminosilicate, a molar ratio of silicon to titanium ([Si]/[Ti]) is preferably in a range of 0.1 to 400, more preferably in the range of 50 to 300, further preferably in the range of 100 to 280, and most preferably in the range of 150 to 260.

When using a compound that easily crystallizes itself, such as TiCl₃, there is a possibility that the element is introduced into an aluminosilicate surface in a cluster form or crystal form. In this case, the [Si]/[Ti] ratio tends to be small because the apparent element content may increase. The [Si]/[Ti] ratio in such a case is preferably in the range of 0.5 to 30. The lower limit value thereof is more preferably 1 and further preferably 1.2. On the other hand, the upper limit value is, more preferably 20 and further preferably 15.

The aluminum content of the modified aluminosilicate of the present invention is not particularly limited, but a molar ratio of silicon to aluminum ([Si]/[Al]) is preferably in a range of 5 to 100,000, more preferably in the range of 10 to 10,000, and further preferably in the range of 15 to 1000.

The modified aluminosilicate of the present invention is preferably characterized by absorption in a specific wavelength region in UV-visible absorption spectrum measurements.

It is preferred that the modified aluminosilicate according to the present invention contains an element selected from the group consisting of elements in Group 4 and Group 5 of the Periodic Table, and usually has an absorbance at 300 nm (A[300]) in an UV-visible absorption spectrum of 1.0 or more. Specific examples and suitable examples of the elements selected from elements in Group 4 and Group 5 contained in the modified aluminosilicate of the present invention are the same as the contents in the above "Fourth step" section.

Moreover, when the element selected from the group consisting of elements in Group 4 and Group 5 of the Periodic Table is contained in a quadrivalent or pentavalent aspect in the modified aluminosilicate that meets the preferable XRD requirements as described above, or when a tetravalent or pentavalent compound of an element selected from the group consisting of elements in Group 4 and Group 5 of the Periodic Table is used in the fourth step, the A[300] preferably has a value exceeding 0.20 (hereinafter also referred to as "specific requirement 0"). Furthermore, for example, when the specific requirement described below (A[300]/A[210] is 0.045 or more and 0.070 or less: hereinafter also referred to as "specific requirement 1") is met, the A[300] is preferably 0.07 to 0.20, more preferably 0.07 to 0.15, and particularly preferably 0.07 to 0.10.

When the A[300] is less than 1.0, or less than 0.20 in the case of the specific requirement 0, or further less than 0.07 when the specific requirement 1 is met, selectivity of an aromatic dihydroxide compound tends to be relatively low upon producing an aromatic dihydroxide compound by a reaction between phenols and hydrogen peroxide. Incidentally, methods used to measure an absorbance are not particularly limited, and it may be the results measured by either a transmission method or reflection method. In the case of measurement by the reflection method, reflected light may include diffuse reflected light as well as normal reflected light, but for the sake of convenience, calculation is carried out based on the assumption that all light is normal reflected light.

The detailed mechanism thereof is unknown, however, the present inventors conjecture as follows (hereinafter, as the modified aluminosilicate, an example of a crystalline porous aluminotitanosilicate in which the element selected from the group consisting of elements in Group 4 and Group 5 is titanium, as described above, will be described.).

Aluminum and titanium are presumed to be present on a framework surface portion of the crystalline porous aluminotitanosilicate. They are also presumed to be preferably present mainly in an inside of concave portions such as pores of the crystalline porous aluminotitanosilicate. Herein, for example, in the case of an aspect that is considered to include only titanium produced by a method using gaseous titanium tetrachloride at elevated temperature as described in Patent Literature 3, and completely incorporated without defects as a framework of a crystal structure, an absorbance in the vicinity of 300 nm in an UV-visible absorption spectrum is conjectured unlikely to be 1.0 or more, or exceeding 0.2 of the specific requirement 0 described above. Within such a range, it is surmised that the condition is such that unstable titanium is present in a slight amount. In other words, the crystalline porous aluminotitanosilicate that meets the requirements of the present invention is presumed to have titanium with an unstable structure in large amount due to incomplete incorporation into its basic framework structure. It is conjectured that such titanium species greatly contribute to an aromatic dihydroxide compound formation reaction, and when an oxidation reaction of phenols with hydrogen peroxide proceeds, formation of 1,2-hydroxide compound (for example, catechol) which is thought to be three-dimensionally disadvantageous due to instability thereof, and formation of benzoquinone that is a form in which the reaction has further proceeded, are inhibited, making it possible for a 1,4-type aromatic dihydroxide compound to be produced with high selectivity.

From the viewpoint of higher selectivity of an aromatic dihydroxide compound, the lower limit value of A[300] of the modified aluminosilicate is preferably 1.5 and more preferably 1.8. In the case of the specific requirement 0, the lower limit value of A[300] is more preferably 0.08, further preferably 0.25, and particularly preferably 0.30. On the other hand, there is no essential significance in setting the upper limit value of the A[300], but the upper limit value thereof is preferably 15 and more preferably 10. The A[300] is also 0.2 or less in ordinary highly crystalline porous aluminotitanosilicate.

A ratio of an absorbance A[300] to an absorbance at 210 nm (A[210]) (A[300]/A[210]) in an UV-visible absorption spectrum of a modified aluminosilicate is more preferably 0.5 or more, further preferably 0.6 or more, and particularly preferably 0.8 or more. The "A[300]/A[210]" in the case of the specific requirement 0 is preferably a value exceeding 0.10. The lower limit value thereof is more preferably 0.11. For example, when the A[300] is a small value less than 0.20, on the other hand, the A[300]/A[210] may preferably be in a range of 0.045 to 0.070. It is considered that, for example, when a framework of a crystal structure contains titanium incorporated therein without defects, the A[210] is relatively higher compared to the A[300], as a result of which in an ordinary crystalline porous aluminotitanosilicate, the A[300]/A[210] usually tends to be 0.1 or lower, but when it is 0.045 or higher, a slight amount of titanium with a special unstable structure is contained, and this titanium may demonstrate an activation effect such as singly or affecting other titanium. Such unstable titanium that may be present in a slight amount has high reactive activity and high structural selectivity, and may demonstrate suitable performance such as high activity, a high yield, and relatively high regioselectivity for production of, for example, the hydroquinone which will be described below.

There is not particular significance in the upper limit value of the A[300]/A[210], but it is more preferably 1.5 and further preferably 1.0. The upper limit value when the unstable titanium is considered to be present in a slight amount as described above is preferably 0.070.

Incidentally, the UV-visible absorption spectrum can be measured by a conventional method. Examples thereof can include the following method.

"A method for placing 0.1 g of a solid aluminosilicate sample in a cell with an optical path length of 10 mm and making measurement in a wavelength range of 200 to 800 nm using a Shimadzu UV-2550 UV-visible spectrophotometer."

Incidentally, the solid aluminosilicate sample is preferably used after being sufficiently dried.

The modified aluminosilicate obtained by the production method of the present invention can be used as a catalyst for producing an aromatic polyhydroxide compound, which is one embodiment of the present invention.

### <Method for producing aromatic polyhydroxide compound>

One embodiment of the present invention is a method for producing an aromatic polyhydroxide compound, which includes a step of reacting an aromatic hydroxide with hydroperoxide in the presence of a catalyst for producing an aromatic polyhydroxide compound, the catalyst containing the modified aluminosilicate.

Examples of the aromatic hydroxides can include, for example, in addition to the phenols described below, compounds having a structure in which one hydroxyl group is mainly bonded to a phenyl framework portion, such as hydroxynaphthalene and its derivative, hydroxyanthracene and its derivative, and hydroxyfluorene and its derivative. Phenols are preferred.

Examples of the hydroperoxides can include, for example, hydrogen peroxide and compounds in which one hydrogen of hydrogen peroxide is substituted with, for example, an aliphatic or aromatic hydrocarbon group, and a heteroatom-containing hydrocarbon group, such as butyl hydroperoxide and cumene hydroperoxide. The hydroperoxide is preferably hydrogen peroxide, and in more detail, hydrogen peroxide water is a preferred aspect.

A method for producing an aromatic polyhydroxide will be described below using a method for producing an aromatic dihydroxide compound as an example. In the presence of the modified aluminosilicate of the present invention, for example, phenols are reacted with hydrogen peroxide to be able to produce an aromatic dihydroxide compound with high selectivity.

The aforementioned phenols refer to unsubstituted phenol and substituted phenol. Here, examples of the substituted phenols include, for example, alkyl phenols substituted with linear or branched alkyl groups having 1 to 6 carbon atoms, such as a methyl group, ethyl group, isopropyl group, butyl group, or hexyl group, or cycloalkyl group.

Examples of phenols include, for example, phenol, 2-methylphenol, 3-methylphenol, 2,6-dimethylphenol, 2,3,5-trimethylphenol, 2-ethylphenol, 3-isopropylphenol, 2-butylphenol, and 2-cyclohexylphenol, with phenol being preferred among these. Note, however, when the phenols have substituents at both the 2-position and 6-position thereof, a formed product is only a hydroquinone derivative.

Examples of the aromatic dihydroxide compounds that are reaction products include, for example, hydroquinones (substituted or unsubstituted hydroquinones) and catechols (substituted or unsubstituted catechols), and specific examples thereof include, for example, hydroquinone, catechol, 2-methylhydroquinone, 3-methylcatechol, 4-methylcatechol, 3-methylhydroquinone, 1,4-dimethylhydroquinone, 1,4-dimethylcatechol, 3,5-dimethylcatechol, 2,3-dimethylhydroquinone, and 2,3-dimethylcatechol.

The modified aluminosilicate obtained in the present invention is used as a catalyst when producing aromatic dihydroxide compounds. Various methods such as fixed bed, fluidized bed, suspension bed, and shelf-stage fixed bed, may be employed as a method of loading a catalyst, and any one of these methods may be carried out. The catalyst may be used as is, or may be molded according to a filling method of the catalyst and used. As methods for molding a catalyst, for example, extrusion molding, tableting molding, rolling granulation, and spray granulation are generally employed. When using the catalyst by a method of fixed bed, extrusion molding or tablet molding is preferred. For the suspension bed method, spray granulation is preferred. After spray granulation, drying and calcination may be carried out. An average particle size of catalysts which underwent the spray granulation is preferably in a range of 0.1 µm to 1,000 µm and more preferably in a range of 5 µm to 100 µm. The average particle size of 0.1 µm or larger facilitates handling such as catalyst filtration, which is preferred, and the average particle size of 1,000 µm or smaller provides a catalyst with favorable performance and strength, which is preferred.

The amount of the catalyst used is preferably 0.1 to 30% by mass and more preferably 0.4 to 20% by mass by outer percentage, relative to the total mass of a reaction liquid (the total mass of liquid components in a reaction system, not including the mass of fixed component such as a catalysts). The amount of 0.1% by mass or more allows a reaction to complete in a shorter time, improving productivity, which is preferred. The amount of 30% by mass or less results in a small amount of catalyst separated and recovered, which is preferred.

When the modified aluminosilicate of the present invention is used as a catalyst for a production method of aromatic dihydroxide compounds, it can be combined with other components. Examples thereof include the siloxane compound described in Patent Literature 1 and the specific alcohol compound described in Patent Literature 2. Such components are preferably used in a proportion such that the mass thereof is 5 to 90% by mass of the reaction liquid. They are more preferably 8 to 90% by mass.

Further, hydrogen peroxide is preferably 0.01 or more and 1 and less in molar ratio with respect to phenols. Concentrations of hydrogen peroxide to be used are not particularly limited, but an aqueous solution with usual 30% concentration may be used, or hydrogen peroxide water with even higher concentration may be used as it is or diluted with a solvent which is inactive in the reaction system. Examples of solvents used for dilution include, for example, an alcohol and water. Hydrogen peroxide may be added all at once or gradually over time.

A reaction temperature is preferably in a range of 30°C to 130°C and more preferably in a range of 40°C to 100°C. The reaction proceeds at temperature outside this range, however, the range described above is preferable from the viewpoint of improving productivity. Reaction pressure is not particularly limited.

A method of the reaction is not particularly limited, and the reaction may be carried out in a batch, semi-batch, or continuous manner. When the continuous manner is employed, the reaction may be carried out in a homogeneous mixing tank of suspension-bed type, or in a plug flow manner with a fixed-bed distribution type, or a plurality of reactors may be connected in series and/or in parallel. The number of reactors is preferably from one to four from the standpoint of equipment cost. When a plurality of reactors is used, hydrogen peroxide may be separately added to each thereof.

In order to obtain an aromatic dihydroxide compound from a reaction liquid, a reaction liquid or a separated liquid containing a dihydroxide compound after separation of the catalyst may be subjected to purification treatment such as removing unreacted components and by-products. The purification treatment is suitably performed on this separated liquid containing the aromatic dihydroxide compound after separation of the catalyst.

Methods of purification treatments are not particularly limited, and specifically include methods of oil-water separation, extraction, distillation, crystallization, and combinations of these methods. A method and procedure, for example, of the purification treatment are not particularly limited, however, for example, a reaction liquid and a separated liquid containing an aromatic dihydroxide compound after separation of the catalyst can be purified by the following method.

When the reaction liquid is separated into two phases of oil phase and water phase, oil and water can be separated. The oil-water separation removes a water phase with low dihydroxide compound content and recovers an oil phase. In this case, the aromatic dihydroxide compound may be recovered from the separated aqueous phase, for example, by extraction and distillation, or a portion or all thereof may be used for a reaction again. A catalyst separated in the catalyst separation step or a catalyst subjected to drying treatment can be dispersed in the separated aqueous phase and then also be supplied to a reactor. On the other hand, the oil phase preferably further undergoes purification treatment by, for example, extraction, distillation, and crystallization.

For extraction, for example, solvents such as 1-butanol, toluene, isopropyl ether, and methyl isobutyl ketone, are used. When the extraction is combined with oil-water separation, the oil-water separation can be carried out efficiently. The extraction solvent is preferably separated and recovered by a distillation column and recycled for use.

Distillation may be carried out on a reaction liquid immediately after catalyst separation or on an oil phase and a water phase after the aforementioned oil-water separation. Furthermore, the extract may be distilled off. When the reaction liquid immediately after catalyst separation is distilled, a light boiling component such as water or an alcohol is preferably separated. Water and alcohols may be separated in separate distillation columns or in a single distillation column.

After having separated water and alcohols, for example, by the aforementioned oil-water separation, extraction, and distillation operation, phenols may be recovered by the next distillation operation and used again in a reaction. When the recovered phenols contain water that has not been completely separated, the water can be removed by adding isopropyl ether or toluene, by azeotropic distillation.

This azeotropic distillation can also be performed on liquid before recovery of phenols and after water and alcohol separation. The separated water can be used again for a reaction or as wastewater. When the recovered phenols contain impurities such as reaction byproducts other than water, they can also be further separated by distillation operation. In a case in which the impurities are benzoquinones, which are reaction byproducts, they can be supplied to a reactor again together with phenols.

After separation of phenols, a component with a higher boiling point than the aromatic dihydroxide compound can be removed by distillation, and hydroquinones and catechols can be separated by the next distillation operation. The components with a high boiling point, hydroquinones, and catechols can also be separated in a single distillation operation by extracting the hydroquinones from a middle stage of the distillation column.

The obtained hydroquinones and catechols undergo removal of impurities, for example, by distillation and crystallization, if necessary, to be able to improve their purity.

For example, when phenol and hydrogen peroxide are reacted in the presence of the modified aluminosilicate according to the present invention, hydroquinone tends to be produced in a high yield. Hydroquinone tends to be produced with higher selectivity compared to, for example, catechol and benzoquinone. Thus, the modified aluminosilicate according to the present invention can be said to be of high industrial value. It is also possible to produce an aromatic polyhydroxide compound under the same conditions as in the method for producing an aromatic dihydroxide compound as described above.

### Examples

The present invention will be described in more detail below by way of Examples, however, the present invention is not limited in any way to these Examples.

### [XRD measurement method]

X-ray diffraction phenomena of sample crystals were measured by the common methods except for the following conditions.
X-ray diffraction apparatus: Model: MultiFlex manufactured by Rigaku Co., Ltd.
X-ray source: CuKα
Output: 40 kV/40 mA
Divergence slit: 1°
Scattering slit: 1°
Light receiving slit 0.30 mm
2θ: 3 to 50°

### [Measurement method of ultraviolet-visible absorption spectrum]

0.1 g of a solid aluminosilicate sample was placed in a cell with an optical path length of 10 mm, and measurement was made in a wavelength range of 200 to 800 nm using a UV-2550 ultraviolet-visible spectrophotometer manufactured by Shimadzu Corporation by a conventional method to determine absorbance intensity at 300 nm and 210 nm.

### [Example 1]

### [Preparation of crystals (A1)]

### Crystals (A1) having an MSE framework were prepared by the following method.

First, 7.90 g (8 mol/L) of a NaOH aqueous solution, 8.18 g (8 mol/L) of a KOH aqueous solution, 20.46 g of a 40% by weight dimethyldipropylammonium hydroxide aqueous solution, and 33.82 g of colloidal silica (product name: LUDOX (registered trademark) AS-40, manufactured by Sigma-Aldrich Co. LLC. (SiO₂ content percentage: 40% by mass)) were fed in a container and stirred at 80°C to obtain a sol-like substance. A stirring time was then adjusted to obtain an HMR value of 3.20. At this time, the raw material was solidified.

Next, 6.91 g of a FAU type zeolite (product name: HSZ-HUA350, manufactured by TOSOH CORPORATION) was added to the resulting mass and mixed using a mortar. Thereafter the mixture was then placed in an autoclave and heated at 160°C for 68 hours. The cooled mixture was filtered, washed with water, and vacuum dried at 70°C for 2 hours to obtain crystals (A1). The highest diffraction peak measured by XRD was observed at 21.66°.

### [First step: Preparation of aluminosilicate]

Next, 7.81 g (8 mol/L) of a NaOH aqueous solution, 8.09 g (8 mol/L) of a KOH aqueous solution, 24.36 g of a 40% by weight dimethyldipropylammonium hydroxide aqueous solution, and 36.13 g of colloidal silica (product name: LUDOX (registered trademark) AS-40, manufactured by Sigma-Aldrich Co. LLC. (SiO₂ content percentage: 40% by mass)) were fed in a container, stirred at 80°C to obtain a sol-like material, and 0.79 g of the crystals (A1) and 6.78 g of a FAU type zeolite (product name: HSZ-HUA350, manufactured by TOSOH CORPORATION) were added and mixed. A stirring time was then adjusted to obtain an HMR value of 4.00. The contents were heated at 165°C for 40 hours using the same autoclave as above. Thereafter, the cooled mixture was filtered, washed with water, and dried under vacuum at 70°C for 2 hours to obtain crystals (1-0).

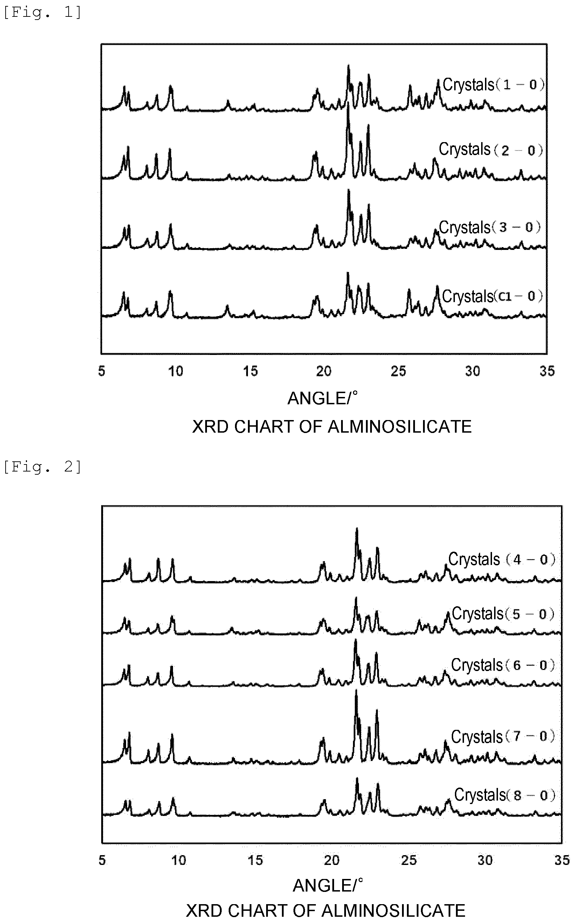

The highest peak in a 17.5 to 35° region measured by XRD of the crystals (1-0) was observed at 21.6°, and the intensity ratio thereof with the highest peak observed at 25.78° in a 25.0 to 27.0° region was 1.73. The XRD chart is shown in FIG 1. The crystals (1-0) were calcinated at 550°C for 10 hours to obtain crystals (1-1).

### [Second step: Contact with acid, third step: Primary calcination]

2 g of the crystals (1-1) prepared above and 80 g of 65% nitric acid were fed in a container and mixed, and then the mixture was fed in an autoclave and heated at 148°C for 24 hours. The cooled mixture was filtered, washed with water, air dried for 1 day, and then underwent primary calcination at 600°C for 2 hours to obtain crystals (1-2).

### [Fourth step: Contact with titanium compound, secondary calcination]

1 g of the crystals (1-2) prepared above and 5 g of a titanium tetrachloride aqueous solution with a Ti concentration of 16% were fed in a container, and heated and stirred at 40°C for 1 hour using an oil bath. Thereafter, water was added, the obtained mixture was filtered and further washed, and the dried product was subjected to secondary calcination at 650°C for 2 hours to obtain a crystalline porous aluminotitanosilicate (crystal (1-3)).

### [Example 2]

### [Preparation of crystals (A2)]

### Crystals (A2) having an MSE framework were prepared by the following method.

First, 15.60 g (8 mol/L) of a NaOH aqueous solution, 16.22 g (8 mol/L) of a KOH aqueous solution, 40.60 g of a 40% by weight dimethyldipropylammonium hydroxide aqueous solution, and 67.20 g of colloidal silica (product name: LUDOX (registered trademark) AS-40, manufactured by Sigma-Aldrich Co. LLC. (SiO₂ content percentage: 40% by mass)) were fed in a container and stirred at 80°C to obtain a sol-like substance. A stirring time was then adjusted to obtain an HMR value of 3.50. At this time, the raw material was solidified.

Next, 13.56 g of FAU-type zeolite (product name: HSZ-HUA350, manufactured by TOSOH CORPORATION) was added to the resulting mass and mixed using a mortar. The mixture was then placed in an autoclave and heated at 165°C for 40 hours. The cooled mixture was filtered, washed with water, and vacuum dried at 70°C for 2 hours to obtain crystals (A2). The highest diffraction peak measured by XRD was observed at 21.60°.

### [First step: Preparation of aluminosilicate]

Next, 7.78 g (8 mol/L) of a NaOH aqueous solution, 8.17 g (8 mol/L) of a KOH aqueous solution, 24.34 g of a 40% by weight dimethyldipropylammonium hydroxide aqueous solution, and 35.33 g of colloidal silica (product name: LUDOX (registered trademark) AS-40, manufactured by Sigma-Aldrich Co. LLC. (SiO₂ content percentage: 40% by mass)) were fed in a container, stirred at 80°C to obtain a sol-like material, and 0.79 g of the crystals (A2) and 6.78 g of a FAU type zeolite (product name: HSZ-HUA350, manufactured by TOSOH CORPORATION) were added and mixed. A stirring time was then adjusted to obtain an HMR value of 4.80. The mixture was then placed in an autoclave and heated at 160°C for 37 hours. Thereafter, the cooled mixture was filtered, washed with water, and dried under vacuum at 70°C for 2 hours to obtain crystals (2-0).

The highest peak in the 17.5 to 35° region measured by XRD of the crystals (2-0) was observed at 21.6°, and the intensity ratio thereof with the highest peak observed at 26.08 in the 25.0 to 27.0° region was 4.30. The XRD chart is shown in FIG 1.

The crystals (2-0) were calcinated at 550°C for 10 hours to obtain a crystal (2-1).

### [Second step: Contact with acid, third step: Primary calcination]

Crystals (2-2) were obtained in the same manner as in Example 1 except that the crystals (2-1) were used instead of the crystals (1-1).

### [Fourth step: Contact with titanium compound, secondary calcination]

Crystals (2-3) were obtained in the same manner as in Example 1 except that the crystals (2-2) were used instead of the crystals (1-2).

### [Example 3]

### [Preparation of crystal (A3)]

Crystals (A3) having an MSE framework were prepared by the following method.

First, 15.62 g (8 mol/L) of a NaOH aqueous solution, 16.22 g (8 mol/L) of a KOH aqueous solution, 40.60 g of a 40% by weight dimethyldipropylammonium hydroxide aqueous solution, and 67.20 g of colloidal silica (product name: LUDOX (registered trademark) AS-40, manufactured by Sigma-Aldrich Co. LLC. (SiO₂ content percentage: 40% by mass)) were fed in a container and stirred at 80°C to obtain a sol-like substance.

Next, 13.56 g of FAU-type zeolite (product name: HSZ-HUA350, manufactured by TOSOH CORPORATION) was added to the resulting sol-like substance and mixed. A stirring time was then adjusted to obtain an HMR value of 4.00. The contents were heated at 165°C for 40 hours using the same autoclave as above. The cooled mixture was filtered, washed with water, and dried under vacuum at 70°C for 2 hours to obtain crystals (A3). The highest diffraction peak measured by XRD was observed at 21.66°.

### [First step: Preparation of aluminosilicate]

Next, 7.81 g (8 mol/L) of a NaOH aqueous solution, 8.14 g (8 mol/L) of a KOH aqueous solution, 24.39 g of a 40% by weight dimethyldipropylammonium hydroxide aqueous solution, and 36.14 g of colloidal silica (product name: LUDOX (registered trademark) AS-40, manufactured by Sigma-Aldrich Co. LLC. (SiO₂ content percentage: 40% by mass)) were fed in a container, stirred at 80°C to obtain a sol-like material, and 0.79 g of the crystals (A3) and 6.78 g of a FAU type zeolite (product name: HSZ-HUA350, manufactured by TOSOH CORPORATION) were added and mixed. A stirring time was then adjusted to obtain an HMR value of 5.50. The mixture was then placed in an autoclave and heated at 160°C for 50 hours. Thereafter, the cooled mixture was filtered, washed with water, and dried under vacuum at 70°C for 2 hours to obtain crystals (3-0).

The highest peak in the 17.5 to 35° region measured by XRD of the crystals (3-0) was observed at 21.64°, and the intensity ratio thereof with the highest peak observed at 26.14 in the 25.0 to 27.0° region was 4.125. The XRD chart is shown in FIG 1.

The crystals (3-0) were calcinated at 550°C for 10 hours to obtain crystals (3-1).

### [Second step: Contact with acid, third step: Primary calcination]

Crystals (3-2) were obtained in the same manner as in Example 1 except that the crystals (3-1) were used instead of the crystals (1-1).

### [Fourth step: Contact with titanium compound, secondary calcination]

Crystals (3-3) were obtained in the same manner as in Example 1 except that the crystals (3-2) were used instead of the crystals (1-2).

### [Comparative Example]

### [Preparation of crystals (A-c1)]

Crystals (A-c1) were obtained in the same manner as in the preparation of the crystals (A1) in Example 1.

### [Preparation of aluminosilicate]

Next, 7.81 g (8 mol/L) of a NaOH aqueous solution, 8.11 g (8 mol/L) of a KOH aqueous solution, 20.30 g of a 40% by weight dimethyldipropylammonium hydroxide aqueous solution, and 32.68 g of colloidal silica (product name: LUDOX (registered trademark) AS-40, manufactured by Sigma-Aldrich Co. LLC. (SiO₂ content percentage: 40% by mass)) were fed in a container, and stirred at 80°C and further stirred for a longer time than in Example 1 followed by dehydration until the HMR reached 3.50. At this time, the mixture was in the state of solidified mass, and therefore pulverization was determined to be necessary in order for the mixture to react with, for example, zeolite in the next step. For this reason, to the raw material mixture were added 0.79 g of the above crystals (A-c1) and 6.78 g of a FAU type zeolite (product name: HSZ-HUA350, manufactured by TOSOH CORPORATION), and the mixture was pulverized in a mortar, and mixed thoroughly, and then fed in an autoclave and heated at 160°C for 55 hours. Thereafter, the cooled mixture was filtered, washed with water, and dried under vacuum at 70°C for 2 hours to obtain crystals (C1-0).

The highest peak in the 17.5 to 35° region measured by XRD of the crystals (C1-0) was observed at 21.58°, and the intensity ratio thereof with the highest peak observed at 25.72 in the 25.0 to 27.0° region was 1.54. The XRD chart is shown in FIG 1.

The crystals (C1-0) were calcinated at 550°C for 10 hours to obtain crystals (C1-1).

### [Contact with acid, primary calcination]

Crystals (C1-2) were obtained in the same manner as in Example 1 except that the crystals (C1-1) were used instead of the crystals (1-1).

### [Contact with titanium compound, secondary calcination]

Crystals (C1-3) were obtained in the same manner as in Example 1 except that the crystals (C1-2) were used instead of the crystals (1-2).

### [Example 4]

### [First step: Preparation of aluminosilicate]

7.81 g (8 mol/L) of a NaOH aqueous solution, 8.09 g (8 mol/L) of a KOH aqueous solution, 20.3 g of a 40% by weight dimethyldipropylammonium hydroxide aqueous solution, and 31.8 g of colloidal silica (product name: LUDOX (registered trademark) AS-40, manufactured by Sigma-Aldrich Co. LLC. (SiO₂ content percentage: 40% by mass)) were fed in a container, and stirred at 80°C to obtain a sol-like substance, and 6.78 g of a FAU type zeolite (product name: HSZ-HUA350, manufactured by TOSOH CORPORATION) were added and mixed. A stirring time was then adjusted to obtain an HMR value of 4.00. The contents were heated at 165°C for 40 hours using the same autoclave as above. The cooled mixture was then filtered, washed with water, and vacuum dried at 70°C for 2 hours to obtain crystals (4-0).

The highest peak in the 17.5 to 35° region measured by XRD of the crystals (4-0) was observed at 21.6°, and the intensity ratio thereof with the highest peak observed at 25.78° in the 25.0 to 27.0° region was 4.39. The XRD chart is shown in FIG 2. The crystals (4-0) were calcinated at 550°C for 10 hours to obtain crystals (4-1).

### [Second step: Contact with acid, third step: Primary calcination]

2 g of the crystals (4-1) prepared above and 80 g of 65% nitric acid were fed in a container and mixed, and then the mixture was fed in an autoclave and heated at 148°C for 24 hours. The cooled mixture was filtered, washed with water, air dried for 1 day, and then underwent primary calcination at 600°C for 2 hours to obtain crystals (4-2).

### [Fourth step: Contact with titanium compound, secondary calcination]

1 g of the crystals (4-2) prepared above and 5 g of a titanium tetrachloride aqueous solution with a Ti concentration of 16% were fed in a container, and heated and stirred at 40°C for 1 hour using an oil bath. Thereafter, water was added, the mixture was filtered and washed further, and the dried product was subjected to secondary calcination at 650°C for 2 hours to obtain a crystalline porous aluminotitanosilicate (crystal (4-3)). The A[300] of the crystal (4-3) was 0.755, the A[210] was 2.873, and the value of A[300]/A[210] was 0.246.

### [Example 5]

Crystals (5-0), crystals (5-1), and crystals (5-2) and a crystalline porous aluminotitanosilicate (crystal (5-3)) were obtained in the same manner as in Example 4 except that in the first step, 31.94 g of colloidal silica (product name: LUDOX (registered trademark) AS-40, manufactured by Sigma-Aldrich Co. LLC. (SiO₂ content percentage was 40% by mass)) was used, the stirring time was adjusted so that the HMR value was 5.00, and the contents were heated at 150°C for 100 hours using an autoclave. The XRD chart of the crystals (5-0) is shown in FIG 2. The A[300] of the crystal (5-3) was 0.083, the A[210] was 1.676, and the value of A[300]/A[210] was 0.050.

### [Example 6]

Crystals (6-0), crystals (6-1), and crystals (6-2) and a crystalline porous aluminotitanosilicate (crystal (6-3)) were obtained in the same manner as in Example 4 except that in the first step, 31.8 g of colloidal silica (product name: LUDOX (registered trademark) AS-40, manufactured by Sigma-Aldrich Co. LLC. (SiO₂ content percentage was 40% by mass)) was used, the stirring time was adjusted so that the HMR value was 5.00, and the contents were heated at 150°C for 120 hours using an autoclave. The XRD chart of the crystals (6-0) is shown in FIG 2. The A[300] of the crystal (6-3) was 0.131, the A[210] was 1.975, and the value of A[300]/A[210] was 0.066.

### [Example 7]

### [Preparation of crystals (A7)]

Crystals (A7) having an MSE framework were prepared by the following method.

First, 15.62 g (8 mol/L) of a NaOH aqueous solution, 16.24 g (8 mol/L) of a KOH aqueous solution, 40.60 g of a 40% by weight dimethyldipropylammonium hydroxide aqueous solution, and 67.23 g of colloidal silica (product name: LUDOX (registered trademark) AS-40, manufactured by Sigma-Aldrich Co. LLC. (SiO₂ content percentage was 40% by mass)) were fed in a container and stirred at 80°C to obtain a sol-like substance.

Next, 13.56 g of FAU-type zeolite (product name: HSZ-HUA350, manufactured by TOSOH CORPORATION) was added to the resulting sol-like substance and mixed. A stirring time was then adjusted to obtain an HMR value of 4.00. The contents were heated at 165°C for 40 hours using the same autoclave as above. The cooled mixture was filtered, washed with water, and dried under vacuum at 70°C for 2 hours to obtain crystals (A7). The highest diffraction peak measured by XRD was observed at 21.58°.

### [First step: Preparation of aluminosilicate]

Next, 7.82 g (8 mol/L) of a NaOH aqueous solution, 8.17 g (8 mol/L) of a KOH aqueous solution, 24.36 g of a 40% by weight dimethyldipropylammonium hydroxide aqueous solution, and 35.38 g of colloidal silica (product name: LUDOX (registered trademark) AS-40, manufactured by Sigma-Aldrich Co. LLC. (SiO₂ content percentage was 40% by mass)) were fed in a container, and stirred at 80°C to obtain a sol-like substance, and 0.79 g of the crystals (A7) and 6.79 g of a FAU type zeolite (product name: HSZ-HUA350, manufactured by TOSOH CORPORATION) were added and mixed.

A stirring time was then adjusted to obtain an HMR value of 6.50 at this stage. Thereafter, the mixture was fed in an autoclave and heated at 150°C for 68 hours. Then, the cooled mixture was then filtered, washed with water, and vacuum dried at 70°C for 2 hours to obtain crystals (7-0).

The highest peak in the 17.5 to 35° region measured by XRD of the crystals (7-0) was observed at 21.58°, and the intensity ratio thereof with the highest peak observed at 26.08 in the 25.0 to 27.0° region was 4.490. The XRD chart is shown in FIG 2. The above crystals (7-0) were calcined at 550°C for 10 hours to obtain crystals (7-1).

### [Second step: Contact with acid, third step: Primary calcination]

Crystals (7-2) were obtained in the same manner as in Example 1 except that the crystals (7-1) were used instead of the crystals (1-1).

### [Fourth step: Contact with titanium compound, secondary calcination]

Crystals (7-3) were obtained in the same manner as in Example 1 except that the crystals (7-2) were used instead of the crystals (1-2). The A[300] of the crystals (7-3) was 0.102, the A[210] was 2.215, and the value of A[300]/A[210] was 0.046.

### [Example 8]

### [First step: Preparation of aluminosilicate]

7.81 g (8 mol/L) of a NaOH aqueous solution, 8.11 g (8 mol/L) of a KOH aqueous solution, 20.35 g of a 40% by weight dimethyldipropylammonium hydroxide aqueous solution, and 33.57 g of colloidal silica (product name: LUDOX (registered trademark) AS-40, manufactured by Sigma-Aldrich Co. LLC. (SiO₂ content percentage was 40% by mass)) were fed in a container, and stirred at 80°C to obtain a sol-like substance, and 6.78 g of a FAU type zeolite (product name: HSZ-HUA350, manufactured by TOSOH CORPORATION) were added and mixed. A stirring time was then adjusted to obtain an HMR value of 4.00 at this stage. The contents were heated at 150°C for 90 hours using the same autoclave as above. Thereafter, the cooled mixture was then filtered, washed with water, and vacuum dried at 70°C for 2 hours to obtain crystals (8-0).

The highest peak in the 17.5 to 35° region measured by XRD of the crystals (8-0) was observed at 21.64°, and the intensity ratio thereof with the highest peak observed at 25.76° in the 25.0 to 27.0° region was 3.54. The XRD chart is shown in FIG 2. The above crystals (8-0) were calcined at 550°C for 10 hours to obtain crystals (8-1).

### [Second step: Contact with acid, third step: Primary calcination]

Crystals (8-2) were obtained in the same manner as in Example 4 except that the crystals (8-1) were used instead of the crystals (4-1).

### [Fourth step: Contact with titanium compound, secondary calcination]

Crystals (8-3) were obtained in the same manner as in Example 5 except that the crystals (8-2) were used instead of the crystals (4-2). The A[300] of the crystals (8-3) was 0.086, the A[210] was 1.682, and the value of A[300]/A[210] was 0.051.

### [Performance evaluation as catalyst]

The performance of the crystalline porous aluminotitanosilicates of Examples 1 to 8 and Comparative Example 1 as catalysts for producing hydroquinone was evaluated below. The results are summarized in Tables 1 and **2.** In the table, HQ denotes hydroquinone and CL denotes catechol. The measurement method and formulae for calculating each value are shown below.

### [Measurement method]

To a flask with an internal volume of 50 ml equipped with a cooler, thermometer, feed pump, and magnetic stirrer tip, were added 0.2 g of each catalyst, 4.2 g of phenol, 3.0 g of t-butyl alcohol, and 6.0 g of water, and the mixture was heated to 50°C in a hot water bath while stirring with a stirrer. 0.5 g of 34% hydrogen peroxide was added dropwise from a feed pump over a period of 10 minutes, and the system was maintained for 60 minutes. After cooling the reaction liquid, the catalyst was filtered off, a portion of the reaction liquid was taken out, and the formed product underwent quantitative determination by gas chromatography.

Analytical conditions of gas chromatography are as follows.
Detector: Hydrogen flame ion detector
Column: DB-5 (Agilent J & W), inner diameter: 0.25 mm, length: 60 m, film thickness: 0.25 µm
Column temperature: The sample was held at 50°C for 10 minutes, raised to 280°C at a rate of temperature rise of 10°C/min.
Inlet: 280°C
Detector temperature: 280°C
Carrier gas; helium
Flow rate: 80ml/min

### (Calculation formula)

The hydroquinone yield was calculated and determined using the following formula. Hydroquinone yield (%) = (hydrogen peroxide utilization efficiency) × (moles of hydroquinone produced)/[(moles of hydroquinone produced) + (moles of catechol produced)] × 100 Hydroquinone/catechol ratio = (moles of hydroquinone produced)/(moles of catechol produced)

**[Table 1]**

| | Relevant to catalyst | | | | | Performance evaluation | |
|---|---|---|---|---|---|---|---|
| | Upon silica preparation | Ti raw material | HMR | Peak intensity ratio | [A300]/ [A210] | HQ yield (/%) | HQ/CL ratio |
| Example 1 | Sol | TiCl₄ solution | 4.0 | 1.73 | 0.457 | 47.1 | 15.0 |
| Example 2 | Sol | TiCl₄ solution | 4.8 | 4.30 | 0.146 | 49.0 | 18.8 |
| Example 3 | Sol | TiCl₄ solution | 5.5 | 4.13 | 0.127 | 55.4 | 28.3 |
| Comparative Example | Gel | TiCl₄ solution | 3.5 | 1.54 | 0.569 | 42.5 | 13.3 |

**[Table 2]**

| | Relevant to catalyst | | | | | Performance evaluation | |
|---|---|---|---|---|---|---|---|
| | Upon silica preparation | Ti raw material | HMR | Peak intensity ratio | [A300]/ [A210] | HQ yield (/%) | HQ/CL ratio |
| Example 4 | Sol | TiCl₄ solution | 4.0 | 4.39 | 0.246 | 56.7 | 15.2 |
| Example 5 | Sol | TiCl₄ solution | 5.0 | 2.38 | 0.050 | 84.0 | 11.9 |
| Example 6 | Sol | TiCl₄ solution | 5.0 | 4.38 | 0.066 | 93.6 | 10.8 |
| Example 7 | Sol | TiCl₄ solution | 6.5 | 4.49 | 0.046 | 88.2 | 10.5 |
| Example 8 | Sol | TiCl₄ solution | 4.0 | 3.54 | 0.051 | 86.8 | 9.7 |

Tables 1 and 2 have demonstrated that the aluminotitanosilicate prepared by the method of the present invention for producing modified aluminosilicates, which meets the stipulation of [1] above, exhibits the high hydroquinone yield and the high hydroquinone selectivity. The sol-like silica can also be used in the course of the method, thereby making it possible to produce the modified aluminosilicate by an industrially advantageous method.

Furthermore, it is found that the use of the modified aluminosilicate within the specific range of the [A300]/[A210] allows the very high HQ yield to be exhibited.

## Claims

1. A method for producing a modified aluminosilicate, comprising
a first step of preparing liquid in which sol-like silica containing water is contacted with a metal compound (AL) containing aluminum and oxygen to obtain an aluminosilicate in which a molar ratio of water to silica (H₂O/SiO₂: HMR) is in a range of 0.1 to 8.0,
a second step of treating the aluminosilicate obtained in the first step with an acid,
a third step of subjecting the treated product obtained in the second step to primary calcination at 550°C to 850°C, and
a fourth step of contacting the calcined product obtained in the third step with liquid containing one or more elements selected from the group consisting of elements in Group 4 and Group 5 of the Periodic Table, followed by carrying out drying and secondary calcination.

2. The method for producing a modified aluminosilicate according to claim 1, wherein the HMR is in the range of 3.0 to 6.6.

3. The method for producing a modified aluminosilicate according to claim 1, wherein the metal compound (AL) containing aluminum and oxygen is a zeolite.

4. The method for producing a modified aluminosilicate according to claim 1, wherein the first step comprises a step of partially removing water.

5. The method for producing a modified aluminosilicate according to claim 1, wherein the first step is a step of preparing liquid in which sol-like silica containing water was contacted with a zeolite, and
then partially removing water in the liquid to obtain an aluminosilicate in which a molar ratio of water to silica (H₂O/SiO₂: HMR) is in a range of 0.1 to 8.0.

6. A method for producing a catalyst for producing an aromatic polyhydroxide compound, the catalyst containing the modified aluminosilicate according to claim 1.

7. A method for producing a catalyst for producing an aromatic polyhydroxide compound, the catalyst containing the modified aluminosilicate according to claim 5.

8. A method for producing an aromatic polyhydroxide compound, comprising a step of reacting an aromatic hydroxide with hydroperoxide in the presence of the catalyst according to claim 6.

9. A method for producing an aromatic polyhydroxide compound, comprising a step of reacting an aromatic hydroxide with hydroperoxide in the presence of the catalyst according to claim 7.

10. A modified aluminosilicate, having a ratio of an absorbance at 300 nm (A[300]) in an ultraviolet-visible absorption spectrum to an absorbance at 210 nm (A[210]) in an ultraviolet-visible absorption spectrum (A[300]/A[210]) of 0.045 or more and 0.070 or less.

## Amended claims

### Amended claims under Art. 19.1 PCT

1. A method for producing a modified aluminosilicate, comprising
a first step of preparing liquid in which sol-like silica containing water is contacted with a metal compound (AL) containing aluminum and oxygen to obtain an aluminosilicate in which a molar ratio of water to silica (H₂O/SiO₂: HMR) is in a range of 0.1 to 8.0,
a second step of treating the aluminosilicate obtained in the first step with an acid,
a third step of subjecting the treated product obtained in the second step to primary calcination at 550°C to 850°C, and
a fourth step of contacting the calcined product obtained in the third step with liquid containing one or more elements selected from the group consisting of elements in Group 4 and Group 5 of the Periodic Table, followed by carrying out drying and secondary calcination.

2. The method for producing a modified aluminosilicate according to claim 1, wherein the HMR is in the range of 3.0 to 6.6.

3. The method for producing a modified aluminosilicate according to claim 1, wherein the metal compound (AL) containing aluminum and oxygen is a zeolite.

4. The method for producing a modified aluminosilicate according to claim 1, wherein the first step comprises a step of partially removing water.

5. The method for producing a modified aluminosilicate according to claim 1, wherein the first step is a step of preparing liquid in which sol-like silica containing water was contacted with a zeolite, and
then partially removing water in the liquid to obtain an aluminosilicate in which a molar ratio of water to silica (H₂O/SiO₂: HMR) is in a range of 0.1 to 8.0.

6. A method for producing a catalyst for producing an aromatic polyhydroxide compound, the catalyst containing the modified aluminosilicate according to claim 1.

7. A method for producing a catalyst for producing an aromatic polyhydroxide compound, the catalyst containing the modified aluminosilicate according to claim 5.

8. A method for producing an aromatic polyhydroxide compound, comprising a step of reacting an aromatic hydroxide with hydroperoxide in the presence of the catalyst according to claim 6.

9. A method for producing an aromatic polyhydroxide compound, comprising a step of reacting an aromatic hydroxide with hydroperoxide in the presence of the catalyst according to claim 7.

10. A modified aluminosilicate, having a ratio of an absorbance at 300 nm (A[300]) in an ultraviolet-visible absorption spectrum to an absorbance at 210 nm (A[210]) in an ultraviolet-visible absorption spectrum (A[300]/A[210]), as measured by filling 0.1 g of a sample in a cell with an optical path length of 10 mm, of 0.045 or more and 0.070 or less.
